# EUROPÄISCHE PATENTSCHRIFT

(11) **EP 2 105 163 B1**
(45) Veröffentlichungstag und Bekanntmachung des Hinweises auf die Patenterteilung: **08.06.2011**
(21) Anmeldenummer: 08005951.2
(22) Anmeldetag: 28.03.2008
(51) Int. Cl.: A61N 2/00

(54) **Magnetwechselfeld-Applikationsvorrichtung zur Aufheizung von magnetischen oder magnetisierbaren Substanzen in biologischem Gewebe**
Varying magnetic field application device for heating magnetic or magnetisable substances in biological tissue
Dispositif d'application de champ magnétique alternatif destiné au chauffage de substances magnétiques ou pouvant être magnétisées dans des tissus biologiques

(43) Veröffentlichungstag der Anmeldung: 30.09.2009
(73) Patentinhaber: MagForce Nanotechnologies AG, 14050 Berlin (DE)
(72) Erfinder: Feucht, Peter, 12247 Berlin-Lankwitz (DE); Brüss, Volker, 12355 Berlin (DE); Jordan, Andreas, Dr., 14129 Berlin (DE)
(74) Vertreter: Liebl, Thomas

(56) Entgegenhaltungen:
- EP-B- 1 102 609
- WO-A-01/56656
- DE-A1- 19 939 001
- US-A- 4 340 038
- US-A- 5 147 284
- US-A1- 2003 045 770

## Beschreibung

Die Erfindung betrifft eine Magnetwechselfeld-Applikationsvorrichtung zur Aufheizung von magnetischen oder magnetisierbaren Substanzen in biologischem Gewebe, insbesondere für die Thermotherapie mit magnetischen Nanopartikeln, nach dem Oberbegriff des Anspruchs 1.

Eine bekannte gattungsgemäße Magnetwechselfeld-Applikationsvorrichtung (EP 1 102 609 B1) besteht aus einem Großapplikator mit einem Magnetjoch und zwei gegenüberliegenden, durch einen Befeldungsspalt beabstandeten Polschuhen am Magnetjoch und mit zwei jeweils einem Polschuh zugeordneten Magnetspulen. Daran ist eine Großapplikator-Steuereinheit zur Einspeisung von Wechselstrom mit bestimmter Amplitude, bestimmter Frequenz und bestimmter Phasenlage angeschlossen zur Erzeugung eines im Befeldungsspalt weitgehend homogenen Magnetwechselfeldes bestimmter Feldstärke. Das zu befeldende biologische Gewebe als Befeldungs-Zielvolumen wird im Befeldungsspalt aufgenommen. Insbesondere ist im Befeldungsspalt ein Patient mit einem zu befeldenden Körperteil als Zielvolumen, beispielsweise mit einer erkrankten Prostata in den Befeldungsspalt einlagerbar.

Im Weiteren wird die erfindungsgemäße Magnetwechselfeld-Applikationsvorrichtung beispielhaft im Wesentlichen anhand eines Prostatakarzinoms beschrieben und erläutert, ohne dass damit eine Beschränkung auf diese Anwendung damit verbunden sein soll, da auch andere Erkrankungen, insbesondere andere Tumore im Oberbauch oder Beckenbereich entsprechend therapierbar sind.

Der bekannte Großapplikator hat einen wirksamen Felddurchmesser des Magnetwechsetfeldes von ca. 300 mm, wobei die Feldstärke bis zu 18 kA/m einstellbar ist. Bei einer Befeldung von Tumoren im Oberbauch oder Beckenbereich, insbesondere eines Prostatakarzinoms eines Patienten wird eine relativ große Körperfläche erfasst, wobei mit der Befeldung große Ringströme induziert werden, die zu einer übermäßigen Erwärmung von Hautoberflächen, von Muskelgewebe und von Knochen, z. B. im Beckenbereich sowie zu unkontrollierbaren Nervenreizungen und damit zu einer erheblichen Belastung eines Patienten führen können. Aufgrund dieser Gegebenheiten kann der bekannte Großapplikator für diese Einsätze nur mit Feldstärken von ca. 4 bis 4,5 kA/m betrieben werden, bei denen die Ringstromwirkungen von einem Patienten üblicherweise noch gut ausgehalten werden können.

Bei einer maximalen Dosierung eines Magnetofluids in einer erkrankten Prostata können bei einer Befeldung nur mit dem Großapplikator dort lediglich Temperaturerhöhungen bis ca. 41 °C oder weniger erreicht werden. Dies reicht zwar für eine Sensibilisierung des Tumors in einer Kombinationsbehandlung in Verbindung mit einer anderen Bestrahlung für einen positiven Therapieeffekt aus. Für eine direkte Zerstörung des Tumorgewebes wäre jedoch eine Temperaturerhöhung auf etwa 45°C oder mehr erforderlich. Bei einem verwendeten 100 kHz-Magnetwechselfeld wäre dazu eine Feldstärke des Großapptikators von etwa 7 kA/m oder mehr erforderlich, die wie oben ausgeführt, von einem Patienten aufgrund der Ringstromwirkungen während der relativ langen erforderlichen Befeldungszeit bis zu etwa einer Stunde praktisch nicht verkraftbar sind.

Aufgabe der Erfindung ist es daher, eine gattungsgemäße Magnetwechselfeld-Applikationsvorrichtung so weiterzubilden, dass eine therapeutisch ausreichende Befeldung relativ kleiner Befeldungszielvolumen, insbesondere relativ kleiner Körperbezirke eines Patienten möglich ist.

Diese Aufgabe wird mit den Merkmalen des Anspruchs 1 gelöst.

Gemäß Anspruch 1 ist im Befeldungsspalt des Großapplikators und im Nahbereich des zu befeldenden biologischen Gewebes als Befeldungs-Zielvolumen, insbesondere an oder in einem Patienten im Nahbereich eines zu befeldenden Körperteil, wie einer erkrankten Prostata, ein Feldkonzentrator angeordnet, der das Magnetwechselfeld des Großapplikators im Zielvolumen konzentriert und dadurch dort lokal verstärkt.

Dadurch ist es vorteilhaft möglich mit relativ geringen Magnetfeldstärken des Großapplikators zu befelden, die keine für einen Patienten unerträglichen Wirbelströme und Ringstrombelastungen entwickeln, wobei dennoch in einem relativ kleinen Zielvolumen, beispielsweise in einer erkrankten Prostata, eine so hohe Feldkonzentration erreicht wird, dass dort eine direkte Zerstörung eines Tumorgewebes bei etwa 45°C oder mehr erreichbar ist.

Konkret wird dazu ein passiver Feldkonzentrator in der Art eines Ferriten vorgeschlagen. Die Konzentrationswirkung und lokale Verstärkung des Magnetwechselfelds des Großapplikators mit einem Ferriten kann vergleichsweise geringer als mit dem nachfolgenden aktiven Feldkonzentrator sein, kann jedoch bei bestimmten Gegebenheiten für Therapiezwecke ausreichend sein. Ein solcher passiver Feldkonzentrator als Ferrit ist besonders einfach und kostengünstig herstellbar. Wie eingangs ausgeführt, besteht die Problematik beim Einsatz des Großapplikators in der unzulässigen Erwärmung eines Patienten durch Ringstromwirkungen und Wirbelstromverluste, wobei diese Probleme durch eine Kombination mit einem geeigneten Feldkonzentrator verringert werden sollen. Der Feldkonzentrator selbst soll aber diese Probleme nicht wieder verstärken. dies wird hier durch die Verwendung eines Ferrits (oxidkeramischer gesinteter Werkstoff) mit der werkstoffspezifischen geringen Leitfähigkeit und den sehr kleinen Wirbelstromverlusten erreicht.

Alternativ dazu wird ein aktiver Feldkonzentrator mit einer Magnetspule als Induktionsspule vorgeschlagen, der zwar aufwendiger ist, jedoch zu einer stärkeren Feldkonzentration und damit zu einer hohen lokalen Verstärkung des Magnetwechselfeldes des Großapplikators verwendbar ist. Damit sind lokale Verstärkungsfaktoren von etwa 3 bis 4 beispielsweise in einem Prostatakarzinom erreichbar. Es ist dazu erforderlich, dass die wenigstens eine Magnetspule des Feldkonzentrators in der Vorrichtung so ausgerichtet ist, dass die Magnetachsen des Feldkonzentrators und des Großapplikators etwa gleich gerichtet sind und dass die Magnetspule mit einem frequenzgleich und phasengleich synchronisierten Wechselstrom, entsprechend dem Wechselstrom des Großapplikators, gespeist wird.

Für eine solche Synchronisation wird mit Anspruch 2 vorgeschlagen, dass die Großapplikator-Steuereinheit und die Feldkonzentrator-Steuereinheit miteinander verbunden oder integriert sind dergestalt, dass beispielsweise auch der Feldkonzentrator direkt durch die Großapplikator-Steuereinheit in Verbindung mit einer Leistungseinheit eingespeist wird. Bevorzugt sind aber auch bei einer solchen direkten Kopplung die Amplituden der Wechselströme jeweils für den Großapplikator und den Feldkonzentrator voneinander unabhängig einstellbar, da insbesondere die maximal zulässige Amplitude am Großapplikator von den individuellen physiologischen Eigenschaften eines Patienten abhängig und verschieden ist.

Alternativ dazu wird für eine Synchronisierung gemäß Anspruch 3 vorgeschlagen, dass die Feldkonzentrator-Steuereinheit und die Großapplikator-Steuereinheit voneinander getrennt sind und unabhängig betrieben werden. Mit der Feldkonzentrator-Steuereinheit wirkt ein Sensor zusammen, der die Frequenz- und Phasenlage des Großapplikator-Magnetwechselfeldes erfasst. Die entsprechenden Werte werden dann in der Feldkonzentrator-Steuereinheit für eine Synchronisation verarbeitet.

In einer besonders bevorzugten Weiterbildung nach Anspruch 4 ist die Magnetspule des aktiven Feldkonzentrators als Flachspule ausgeführt, wobei das zu befeldende Zielvolumen etwa senkrecht zur Flachspulenebene im Bereich der Magnetachse anzuordnen ist. Eine Zylinderspule ist dagegen für einen aktiven Feldkonzentrator wenig geeignet.

Für eine Befeldung eines Tumors im Oberbauch oder Beckenbereich, insbesondere eines Prostatakarzinoms, wird der Nahbereich zum Zielvolumen gemäß Anspruch 5 dadurch hergestellt, dass der Feldkonzentrator als Rektal-applikator ausgebildet ist mit einem flachen länglichen Gehäuse in einer Gestalt und Größe, die dem Rektum eines Patienten als Aufnahmeraum angepasst ist. Ein solches längliches Gehäuse dient als Ummantelung für eine längsgestreckte Flachspule als Magnetspule, so dass deren Magnetachse in etwa senkrecht zur Gehäuseebene verläuft. So eine längsgestreckte Flachspule kann dadurch erhalten werden, dass eine kreisringförmige Flachspule gewickelt wird, die anschließend zu einer längsgestreckten Form gestaucht und gegebenenfalls noch leicht gebogen wird.

Nach Anspruch 6 ist an einem Gehäuselängsende und entsprechend den anatomischen Gegebenheiten abgewinkelt zur Gehäuseebene ein rohrförmiger Einführstutzen mit dem Gehäuse verbunden. Der Rektalapplikator ist mit diesem Einführstutzen in das Rektum einführbar und damit gegebenenfalls in der Lage justierbar und fixierbar. Aufgrund der anatomischen Vorgaben ist das Gehäuse gemäß Anspruch 7 etwa 65 mm bis 70 mm lang, 20 mm hoch und 35 mm breit, mit einem etwa ovalen Querschnitt und gerundeten Schmalflächen. Dabei soll die Fläche in Feldrichtung möglichst groß sein. Je größer diese Fläche ist umso größer ist die verwertbare Reichweite des Magnetfeldes, das die Magnetspule in Richtung des Feldes abgibt. Der Einführstutzen hat gegenüber dem Gehäuse einen kleineren Durchmesser von etwa 10 mm, da der Rohransatz bei der Anwendung des Rectalapplikators im Bereich des Schließmuskels verbleibt und durch seinen geringen Durchmesser dort eine Reizung gemindert wird Weiter soll die Vetrählichkeit nach Anspruch 8 dadurch verbessert werden, dass zwar das Gehäuse und der Einführstutzen formstabil hergestellt sind, jedoch wenigstens das Gehäuse eine Auflage aus weichem Material aufweist.

Nach Anspruch 9 sind in einer vorteilhaften konkreten Ausführungsform die elektrischen Anschlussleitungen zur Magnetspule sowie ein Kühlmittelzulauf und ein Kühlmittelablauf am Einführstutzen angeschlossen und/oder durch diesen in das Gehäuse geführt. Über das Kühlmittel werden sowohl die Magnetspule im Gehäuse als auch die Anschlussleitungen gekühlt.

Nach Anspruch 10 sind im weiteren Verlauf die elektrischen Anschlussleitungen und Kühlmittelschläuche mit möglichst kleinem Querschnitt in einem flexiblen, am Einführstutzen angebrachten Anschlussschlauch aufgenommen. Wesentlich ist dabei, dass durch den flexiblen Anschlussschlauch mit den flexiblen Anschlussleitungen während des relativ langen Befeldungszeitraums möglichst keine Querkräfte auf den Einführstutzen wirken, die die Verträglichkeit verringern und zu einer ungünstigen Dejustierung bezüglich der Feldrichtung des Großapplikators führen könnten.

In einer vorteilhaften Ausführung nach Anspruch 11 sind die elektrischen Anschlussleitungen und ein Kühlmittelzulaufschlauch im Anschlussschlauch und im Einführstutzen geführt, wobei der verbleibende Restquerschnitt des Anschlussschlauchs bzw. des Einführstutzens für den Kühlmittelrücklauf verwendet ist. Damit wird eine einfache Anordnung in Verbindung mit einer wirksamen Kühlung der elektrischen Anschlussleitungen, welche im Rückstrom geführt sind, erreicht.

Mit Anspruch 12 werden klinisch erfolgreich getestete Einstellwerte angegeben dergestalt, dass am Großapplikator eine Feldstärke von ca. 3 kA/m bis 4 kA/m eingestellt wird, wobei in Verbindung insbesondere mit dem vorstehenden Rektalapplikator eine 3-fache bis 4-fache Feldstärkeerhöhung im Zielvolumen, insbesondere in einem Protatakarzinom erreichbar ist.

Gemäß Anspruch 13 wird regelmäßig die Behandlung in einem Großapplikator in Verbindung mit dem aktiven Feldkonzentrator, insbesondere als Rektalapplikator, bei einer zeitgleichen Aktivierung beider Applikatoren erfolgen. Je nach den Gegebenheiten kann auch eine zeitlich versetzte Aktivierung, gegebenenfalls in Verbindung mit nachfolgenden gleichzeitigen Aktivierungen der Einzelapplikatoren zu Therapieerfolgen führen. Gegebenenfalls ist auch ein von einem Großapplikator unabhängiger und separater Einsatz des Rektalapplikators für eine Therapie möglich, insbesondere wenn sich ein Behandlungsgebiet nur etwa 10 bis 20 mm von der Darmwand des Rektums entfernt befindet. Auch eine solche Verwendung soll vom Schutz umfasst ein, wobei für den Rektalapplikator auch als separate Einheit Schutz beansprucht wird.

Bei der Behandlung eines Patienten im Großapplikator ist grundsätzlich ein annähernd homogenes Magnetfeld im Unterleib vom Bauch zum Rücken weisend vorhanden, wobei ein eingeführter Rektalapplikator die Funktion des aktiven Feldkonzentrators im Darm- und Prostatabereich hat. Durch die Anatomie des Menschen, entsprechend der Ausrichtung des Rektums bedingt, ist jedoch die Magnetfeldachse des Rektalapplikators gegenüber der Magnetfeldachse des Großapplikators in Kopfrichtung etwas geneigt, so dass die an sich geforderte genaue, gleiche Ausrichtung der beiden Magnetfeldachsen in Verbindung mit einem Rektalapplikator nicht möglich ist. Dadurch wird die Funktion des Rektalapplikators als Feldkonzentrator zur Feldverstärkung in einer erkrankten Prostata zwar reduziert, jedoch ist bei einer gegenseitigen Neigung der Feldachsen um ca. 20° bis 30° ein damit verbundender Verlust der Feldstärke im Vergleich zu einer maximalen Verstärkung zwischen 6 % und 14 % für die Therapie noch gut ausreichend und akzeptabel.

Bei der Anwendung des Rektalapplikators besteht das grundsätzliche Problem, dass dessen Magnetspule relativ klein ist, und deren Magnetfeld in der dritten Potenz mit dem Abstand abnimmt. Es ist daher anzustreben, möglichst viel von der Feldstärke des Großapplikator einzusetzen indem dessen Feldstärke möglichst hoch eingestellt wird. Wie eingangs erläutert, führt jedoch eine hohe Feldstärkeeinstellung am Großapplikator zu einer unerwünschten Erwärmung des gesamten Patienten, durch die in ihm induzierten Ringströme als Funktion der befeldeten Flächen. Dieses Problem tritt besonders bei Patienten mit größerer Leibesfülle auf. Das Problem vergrößert sich noch dadurch, dass in Folge der Leibesfülle der Befeldungsspalt am Großapplikator weit eingestellt werden muss, was die unerwünschten Ringströme im Patienten zusätzlich vergrößert. Es wird daher mit Anspruch 14 vorgeschlagen, dass insbesondere bei dickleibigen Patienten unmittelbar an der Oberfläche über und/oder unter einem Patienten eine Vorfokussierung des Magnetwechselfeldes durch weitere, entsprechend angeordnete flache Induktionsspulen erfolgt, die entsprechend frequenz- und phasensynchron erregt werden.

Anhand einer Zeichnung wird die Erfindung weiter erläutert.

Es zeigen:
- Fig. 1: eine schematische Schnittansicht durch einen Magnetfeldapplikator als Großapplikator,
- Fig. 2: eine schematische Darstellung eines Magnetwechselfeldes des Großapplikators mit einem Feldkonzentrator,
- Fig. 3: eine schematische Darstellung eines Patienten mit einem Prosta- takarzinom mit eingesetztem Rektalapplikator,
- Fig. 4a, b, c: verschiedene Ansichten eines Rektalapplikators,
- Fig. 5: einen Querschnitt durch den Einführstutzen des Rektalapplikators nach Fig. 4, und
- Fig. 6: ein Blockschaltbild der Steuer- und Leistungselektronik des Rektal- applikators.

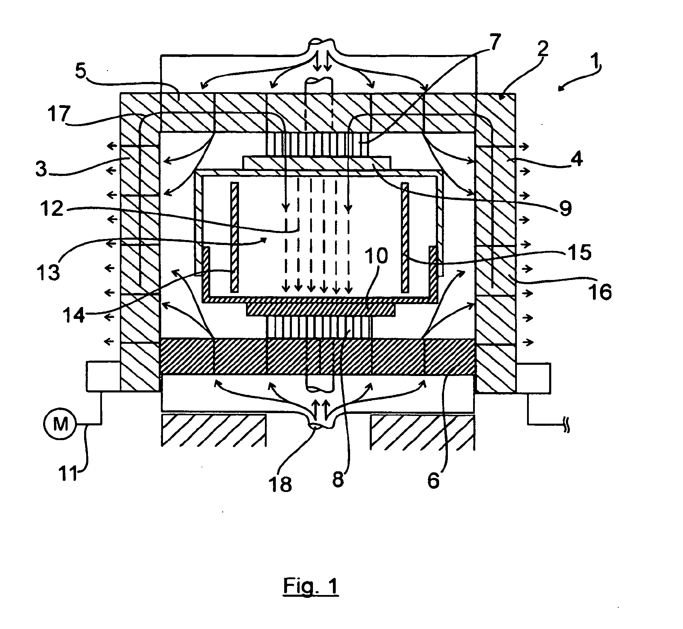

In Fig. 1 ist schematisch ein Magnetfeldapplikator als Großapplikator 1 für die Thermotherapie bzw. Hyperthermie dargestellt, in dem ein Körper, in den eine magnetische oder magnetisierbare Substanz als Magnetofluid eingebracht ist, befeldbar ist.

Der Großapplikator 1 umfasst ein Magnetjoch 2, das in einer M-Form als Dreischenkelanordnung ausgebildet ist und zwei beabstandete parallele Vertikaljochteile 3, 4 sowie zwei dazwischen angeschlossene Querjochteile 5, 6 aufweist.

Eine Baueinheit aus einem unteren Querjochteil 6 und diesem zugeordneten unteren Polschuh 8 mit unterer Magnetspule 10 ist ortsfest angebracht. Demgegenüber kann ein Portal aus den beiden Vertikaljochteilen 3, 4, dem angeschlossenen oberen Querjochteil 5 sowie diesem zugeordneten oberen Polschuh 7 mit oberer Magnetspule 9 mittels eines hier schematisch dargestellten selbsthemmenden Spindelantriebs 11 zur Einstellung der Befeldungsspaltweite des Befeldungsspalts 13 verstellt werden. Im Befeldungsspalt 13 kann ein etwa homogenes Magnetwechselfeld 12 mit einer für einen Patienten verträglichen Feldstärke (bei der weiter unten erläuterten Anwendung von ca. bis 4 kA/m) erzeugt werden.

Der Befeldungsspalt 13 ist durch Schottwände 14, 15 begrenzt, die einen Einschubraum für einen Patienten umgrenzen.

Die obere Magnetspule 9 und die untere Magnetspule 10 sind als Scheibenspulen ausgebildet mit einer oder mehreren Windungen, die schneckenförmig verlaufen und aus verseilten Kupferlitzendrähten hergestellt sind.

Das Magnetjoch 2 und die Polschuhe 7, 8 bestehen aus Ferritbausteinen 16 mit dazwischenliegenden Spalten. Am Großapplikator 1 ist ein Kühlgehäuse mit Aussparungen 18 vorgesehen, durch die Kühlluft eingeführt wird, die durch die Spalten am Magnetjoch wieder austritt. Die Ferritbausteine 16 sind aus aneinandergereihten, im Magnetjoch 2 längs der Magnetflussrichtung 17 ausgerichteten Ferritplatten aufgebaut, die quer zur Magnetflussrichtung 17 durch die Kühlungsspalten voneinander getrennt sind.

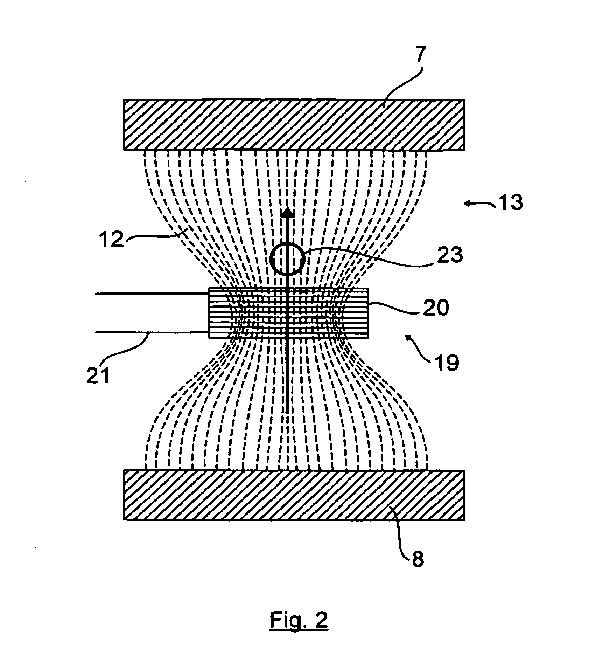

In Fig. 2 ist schematisch der Bereich des Befeldungsspalts 13 zwischen den Polschuhen 7, 8 des Großapplikators dargestellt, mit einem darin angebrachten aktiven Feldkonzentrator 19. Der Feldkonzentrator 19 besteht aus einer Flachspule 20 mit Anschlussleitungen 21, wobei die Magnetachse des Großapplikators und die Magnetachse der Flachspule 20 gleichgerichtet sind und zusammenfallen. Zudem wird die Flachspule 20 mit einem frequenzgleich und phasengleich synchronisierten Wechselstrom entsprechend dem Wechselstrom des Großapplikators 1 gespeist. Dadurch ergibt sich die dargestellte Funktion eines aktiven Feldkonzentrators 19 mit einer Feldverstärkung im Bereich der Flachspule 20. Im Nahbereich der Flachspule 20 ist hier schematisch ein zu befeldendes Körperteil, beispielsweise eine krankhaft vergrößerte Prostata 23, als Zielvolumen gelagert, wo ersichtlich eine Konzentration und lokale Verstärkung des Magnetwechselfeldes des Großapplikators durch den Feldkonzentrator 19 erreicht ist.

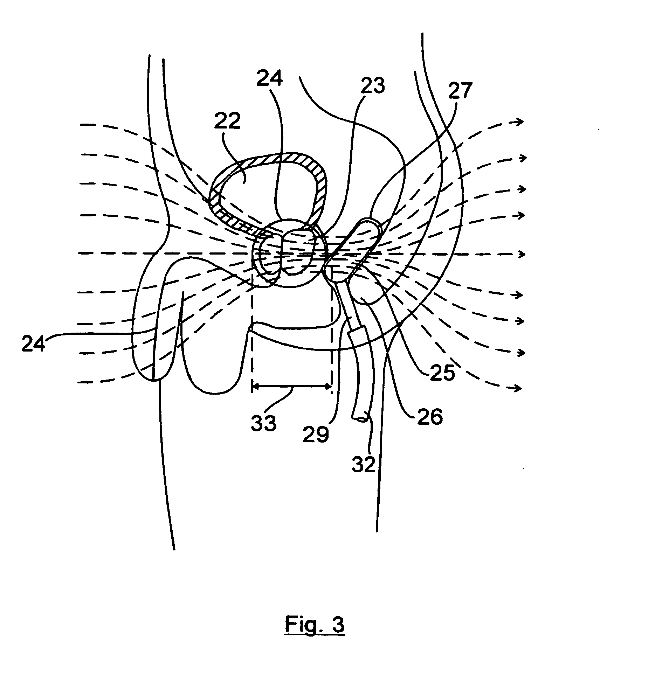

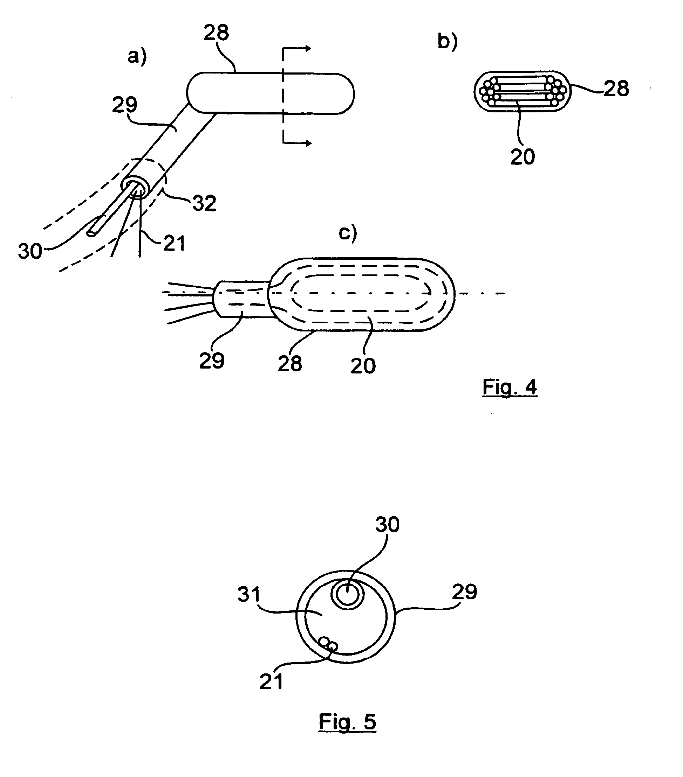

In Fig. 3 ist in konkreterer Weise die Anordnung nach Fig. 2 gezeigt, wobei eine durch eine krankhaft vergrößerte Prostata 23 entsprechend befeldet wird. Dazu ist ein schematischer Schnitt durch einen Patienten im Bereich des Unterleibs gezeigt mit einer Harnblase 22, der am unteren Blasenausgang angeordneten Prostata 23, welche die Harnröhre 24 ringförmig umgibt. Der Bereich der Prostata ist hier das Zielvolumen für die Befeldung und durch einen Kreis 25 in seiner Lage und Größe angegeben. Als aktiver Feldkonzentrator 19 ist hier ein Rektalapplikator 25 am Schließmuskel 26 vorbei in das Rektum 27 eingeführt. Der Rektalapplikator 25 wird näher anhand der Fig. 4 und 5 erläutert:

In Fig. 4a ist eine Seitenansicht, in Fig. 4b ein Querschnitt und in Fig. 4c eine Draufsicht auf den Rektalapplikator 25 gezeigt. Der Rektalapplikator 25 enthält eine Flachspule 20 in länglicher Form, die von einem Gehäuse 28 ummantelt ist. Das Gehäuse ist etwa 70 mm lang, 20 mm hoch und 35 mm breit mit einem etwa ovalen Querschnitt und gerundeten Schmalflächen, wobei diese Größe dem Aufnahmevolumen des Rektums 27 entspricht. An einem Gehäuselängsende und abgewinkelt zur Gehäuseebene ist ein rohrförmiger Einführstutzen 29 angeformt, der wie aus Fig. 3 ersichtlich, entsprechend den anatomischen Gegebenheiten zur Gehäuseebene abgewinkelt liegt. Das Gehäuse 28 kann zudem eine Auflage aus weichem Material aufweisen.

Wie insbesondere der Querschnitt durch den Einführstutzen 29 nach Fig. 5 zeigt, sind die elektrischen Anschlussleitungen 21 und ein Kühlmittelzulaufschlauch 30 im Einführstutzen 29 geführt und angeschlossen, wobei der verbleibende Restquerschnitt 31 als Kühlmittelrücklauf verwendet wird. An den formstabilen Einführstutzen 29 schließt sich ein flexibler Anschlussschlauch 22 an, durch den die elektrischen Anschlussleitungen 21 und der Kühlmittelzulaufschlauch 30 weitergeführt sind.

Aus Fig. 3 ist ersichtlich, dass die effektive Reichweite des Rektalapplikators 25 in seiner Funktion als aktiver Feldkonzentrator den Prostatabereich erfassen muss, entsprechend einer mit dem Doppelpfeil 33 eingezeichneten Reichweite von ca. 70 mm. Weiter ist aus Fig. 3 ersichtlich, dass aufgrund der anatomischen Lage des Rektums 27 die Magnetachse der Flachspule 20 im Rektalapplikator 25 gegenüber der Magnetachse des Großapplikators (verläuft hier horizontal) um einen Winkel nach oben geneigt ist. Dadurch ist die Feldkonzentratorwirkung des Rektalapplikators etwas reduziert gegenüber dem Idealfall gleich ausgerichteter Magnetachsen, jedoch ist die Konzentratorwirkung ausreichend und akzeptabel.

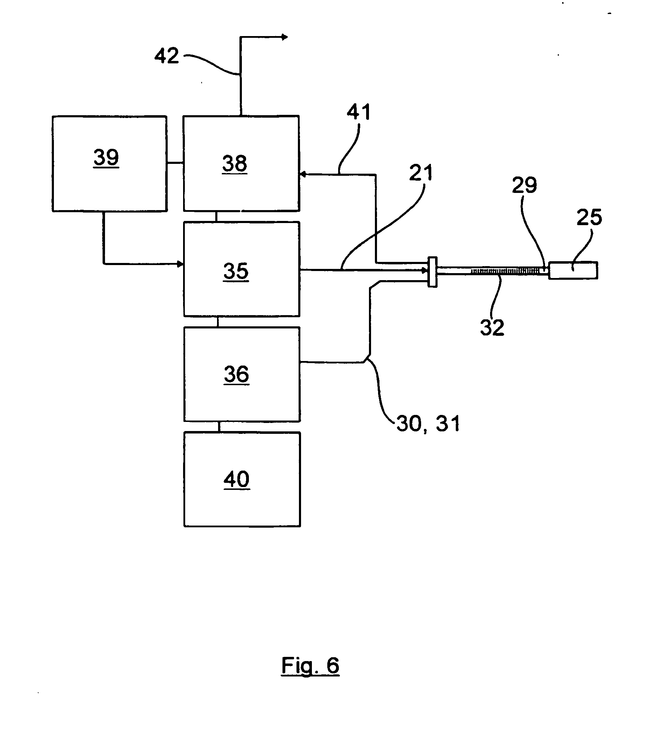

In Fig. 6 ist Blockschaltbild 34 für die Einspeisung und Steuerung des Rektalapplikators 25 dargestellt. Am Einführstutzen 29 ist hier konkret ein Anschlussschlauch 32 mit einem Maßstab für eine Einführjustierung angeschlossen, durch den die elektrischen Anschlussleitungen 21 von einem Leistungsverstärker 35 sowie die Kühlmittelzufuhr und -abfuhr 30, 31 ausgehend von einem Durchlaufthermostat 36 geführt sind. Weiter ist eine Steuereinheit 38 vorgesehen, die mit einer Bedieneinheit 39 insbesondere für eine Leistungseinstellung zusammenwirkt. Zudem ist eine Überwachungseinheit 40 vorgesehen. Vom Rektalapplikator 25 kann eine Signalleitung 41 zurück zur Steuereinheit geführt sein, die mit Sensoren im Bereich des Rektalapplikators 25 verbunden sind. Dies können eine oder mehrere Sensoren sein, die eine Lage erfassen oder die für eine Synchronisation die Frequenz und Phase des Magnetwechselfeldes des Großapplikators erfassen und für einen Abgleich an die Steuereinheit 38 weiterleiten. Die Steuereinheit 38 kann für eine solche Synchronisation aber auch direkt mit einer Steuereinheit des Großapplikators verbunden sein, wie dies schematisch mit der Leitung 42 angedeutet ist.

## Patentansprüche

1. Magntewechselfeld-Applikatlonsvorrichtung zur Aufheizung von magnetischen oder magnetisierbaren Substanzen in biologischem Gewebe, insbesondere für die Thermotherapie mit magnetischen Nanopartikeln, aufweisend
einen Großapplikator (1) mit einem Magnetjoch (2) und zwei gegenüberliegenden, durch einen Befeldungsspalt (13) beabstandeten Polschuhen (7, 8) am Magnetjoch (2), und
mit zwei jeweils einem Polschuh (7, 8) zugeordneten Magnetspulen (9, 10) mit angeschlossener Großapplikator-Steuereinheit zur Einspeisung von Wechselstrom mit bestimmter Amplitude, bestimmter Frequenz und bestimmter Phasenlage zur Erzeugung eines im Befeldungsspalt (13) weitgehend homogenen Magnetwechseifeldes (12) bestimmter Feldstärke,
wobei das zu befeldende biologische Gewebe als Befeldungr-Zielvolumen in den Befeldungsspalt (13) einbringbar ist, insbesondere ein Patient mit einem zu befeldenden Körperteil wie einer erkrankten Prostata (23) in den Befeldungsspalt (13) einlagerbar ist,
**dadurch gekennzeichnet,**
**dass** im Befeldungsspalt (13) im Nahbereich des zu befeldenden biologischen Gewebes als Befeldungs-Zielvolumen, insbesondere an oder in einem Patienten im Nahbereich eines zu befeldenden Körperteils wie einer erkrankten Prostata (23), ein Feldkonzentrator (19) angeordnet ist, der das Magnetwechselfeld (12) des Großapplikators (1) im Zielvolumen konzentriert und **dadurch** dort lokal verstärkt, wobei der Feldkonzentrator ein Ferrit als passiver Feldkonzentrator ist, oder der Feldkonzentrator ein aktiver Feldkonzentrator (19) mit wenigstens einer Magnetspule (20) ist und die wenigstens eine Magnetspule (20) so ausgerichtet ist, dass die Feldlinien des aktiven Feldkonzentrators (19) und des Großapplikators (1) etwa gleichgerichtet sind, sowie eine Feldkonzentrator-Steuereinheit (35, 38) vorgesehen ist, mit der die wenigstens eine Magnetspule (20) mit einem frequenzgleich und phasengleich synchronisierten Wechselstrom entsprechend dem Wechselstrom des Großapplikators (1) gespeist wird.

2. Magnetwechselfeld-Applikationsvorrichtung nach Anspruch 1, **dadurch gekennzeichnet, dass** die Großapplikator-Steuereinheit und die Feldkonzentrator-Steuereinheit (38) für einen aktiven Feldkonzentrator (19) für eine Synchronisation der Frequenz und Phasenlage miteinander verbunden oder ineinander integriert sind, wobei bevorzugt die Amplituden der Wechselströme jeweils für den Großapplikator und den Feldkonzentrator voneinander unabhängig einstellbar sind.

3. Magnetwechselfeld-Applikationsvorrichtung nach Anspruch 1, **dadurch gekennzeichnet, dass** die Feldkonzentrator-Steuereinheit (38) als von der Großapplikator-Steuereinheit unabhängige Einheit wenigstens einen Sensor zur Erfassung und für eine Synchronisation der Frequenz und Phasenlage mit dem Magnetwechselfeld des Großapptikators (1) aufweist.

4. Magnetwechseifeld-Applikationsvorrichtung nach einem der Ansprüche 1 bis 3, **dadurch gekennzeichnet, dass** die wenigstens eine Magnetspule des aktiven Feldkonzentrators (19) eine Flachspule (20) ist.

5. Magnetwechselfeld-Applikationsvorrichtung nach einem der Ansprüche 1 bis 4, **dadurch gekennzeichnet, dass** der aktive Feldkonzentrator (19) als Rektal-Applikator (25) ausgebildet ist, mit einem flachen, länglichen Gehäuse (28) in einer Gestalt und Größe, die dem Rectum (27) eines Patienten als Aufnahmeraum angepasst ist, und
dass im Gehäuse (28) eine entsprechend längsgestreckte Flachspule (20) als Magnetspule enthalten ist, so dass deren Feldrichtung etwa senkrecht zur Gehäuseebene verläuft.

6. Magnetwechselfeld-Applikationsvorrichtung nach Anspruch 5, **dadurch gekennzeichnet, dass** an einem Gehäuselängsende und abgewinkelt zur Gehäuseebene ein rohrförmiger Einführstutzen (29) mit dem Gehäuse (28) verbunden ist, wobei der Rektal-Applikator (25) mit dem Einführstutzen (29) in das Rectum (27) einführbar und damit in seiner Lage justierbar und fixierbar ist.

7. Magnetwechselfeld-Applikationsvorrichtung nach Anspruch 6, **dadurch gekennzeichnet, dass** das Gehäuse (28) etwa 65 mm bis 70 mm lang, etwa 20 mm hoch und etwa 35 mm breit ist mit einem etwa ovalen Querschnitt und gerundeten Schmalflächen,
dass der Einführstutzen (29) einen dagegen kleineren Durchmesser von etwa 10 mm sowie eine Länge von ca. 70 mm bis 100 mm aufweist.

8. Magnetwechselfeld-Applikationsvorrichtung nach Anspruch 7, **dadurch gekennzeichnet, dass** das Gehäuse (28) und der Einführstutzen (29) formstabil hergestellt sind und wenigstens das Gehäuse (28) eine Auflage aus weichem Material aufweist, das sich an die Innenwand des Rectums (27) anpasst.

9. Magnetwechselfeld-Apptikationsvorrichtung nach einem der Ansprüche 5 bis 8, **dadurch gekennzeichnet, dass** die elektrischen Anschlussleitungen (21) zur Magnetspule (20) sowie ein Kühlmittelzulauf (30) und ein Kühlmittelablauf (31) am Einführstutzen (29) angeschlossen und/oder durch diesen in das Gehäuse (28) geführt sind.

10. Magnetwechselfeld-Applikationsvorrichtung nach Anspruch 9, **dadurch gekennzeichnet, dass** die elektrischen Anschlussleitungen (21) und Kühlmittelschläuche (30) in einem flexiblen, am Einführstutzen (29) angebrachten Anschlussschlauch (32) aufgenommen sind.

11. Magnetwechselfeld-Applikationsvorrichtung nach Anspruch 10, **dadurch gekennzeichnet, dass** die elektrischen Anschlussleitungen (21) und ein Kühlmittelzulaufschlauch (30) im Anschlussschlauch (32) und im Einführstutzen (29) geführt sind und dass der verbleibende Restquerschnitt (31) als Kühlmittelrücklauf verwendet ist.

12. Magnetwechselfeld-Applikationsvorrichtung nach einem der Ansprüche 1 bis 11, **dadurch gekennzeichnet, dass** am Großapplikator (1) eine Feldstärke von ca. 3 kA/m bis 4kA/m eingestellt ist und durch den aktiven Feldkonzentrator eine ca. 3 bis 4 fache Feldstärkeerhöhung im Zielvolumen, insbesondere in einer erkrankten Prostata (23) erfolgt.

13. Magnetwechselfeld-Applikationsvorrichtung nach einem der Ansprüche 1 bis 11, **dadurch gekennzeichnet, dass** der aktive Feldkonzentrator (19), insbesondere als Rektalapplikator (25) zeitgleich oder teilweise zeitgleich mit dem Großapplikator oder unabhängig und separat von diesem aktivierbar und einsetzbar ist.

14. Magnetwechselfeld-Applikationsvorrichtung nach einem der Ansprüche 1 bis 13, **dadurch gekennzeichnet, dass** die Vorrichtung zur Vorfokussierung des Magnetwechselfeldes (12) eingerichtet ist, insbesondere bei dickleibigen Patienten unmittelbar an der Oberfläche über und/oder unter einem Patienten, durch weitere dort angeordnete flache Induktionsspulen die entsprechend frequenz- und phasensynchron zum Magnetwechselfeld (12) des Großapplikators (1) erregbar sind.

## Claims

1. Alternating-magnetic-field application device for heating magnetic or magnetizable substances in biological tissue, in particular for thermotherapy using magnetic nanoparticles, with
a large-scale applicator (1) with a magnetic yoke (2) and two opposing pole shoes (7, 8) on the magnetic yoke (2), which shoes are separated by a field-exposure gap (13), and
with two magnetic coils (9, 10), each associated with a pole shoe (7, 8), with an attached large-scale applicator control unit for supplying an alternating current, with a certain amplitude, a certain frequency and a certain phase, for generating an alternating magnetic field (12), which is largely homogenous in the field-exposure gap (13) and has a certain field strength,
wherein, as a field-exposure target volume, the biological tissue to be exposed to the field can be introduced into the field-exposure gap (13); in particular, a patient with a body part to be exposed to the field, such as a diseased prostrate (23), can be placed into the field-exposure gap (13),
**characterized**
**in that** a field concentrator (19) is arranged in the field-exposure gap (13) in the vicinity of the biological tissue to be exposed to the field as a field-exposure target volume; more particularly, it is arranged on or in a patient in the vicinity of a body part to be exposed to the field, such as a diseased prostrate (23), which field concentrator, in the target volume, concentrates the alternating magnetic field (12) from the large-scale applicator (1) and thereby brings about a local increase in the field strength, wherein the field concentrator is a ferrite as a passive field concentrator, or the field concentrator is an active field concentrator (19) with at least one magnetic coil (20), and the at least one magnetic coil (20) is aligned such that the field lines of the active field concentrator (19) and the large-scale applicator (1) are aligned in approximately the same direction, and provision is made for a field-concentrator control unit (35, 38), by means of which the at least one magnetic coil (20) is fed with an alternating current, which is synchronized in terms of frequency and phase, and which corresponds to the alternating current of the large-scale applicator (1).

2. Alternating-magnetic-field application device according to Claim 1, **characterized in that** the large-applicator control unit and the field-concentrator control unit (38) for an active field concentrator (19) are interconnected or integrated in one another for synchronizing the frequency and the phase, wherein the amplitudes of the alternating currents, respectively for the large-scale applicator and the field concentrator, can preferably be set independently of one another.

3. Alternating-magnetic-field application device according to Claim 1, **characterized in that** the field-concentrator control unit (38), as a unit that is independent of the large-scale applicator control device, has at least one sensor for registering the frequency and the phase of the alternating magnetic field of the large-scale applicator (1) and for synchronizing said phase and said frequency thereto.

4. Alternating-magnetic-field application device according to one of Claims 1 to 3, **characterized in that** the at least one magnetic coil of the active field concentrator (19) is a flat coil (20).

5. Alternating-magnetic-field application device according to one of Claims 1 to 4, **characterized in that** the active field concentrator (19) is designed as a rectal applicator (25) with a flat elongate housing (28) in a shape and size that is fitted to the rectum (27) of a patient as a holding space, and
**in that** the housing (28) contains a correspondingly elongated flat coil (20) as a magnetic coil, and so the field direction of said magnetic coil runs approximately perpendicular to the plane of the housing.

6. Alternating-magnetic-field application device according to Claim 5, **characterized in that** a tubular insertion tube (29) is connected to the housing (28) at one longitudinal end of the housing and at an angle to the housing plane, wherein the rectal applicator (25) can be inserted into the rectum (27) with the insertion tube (29) and the position of said applicator can thereby be adjusted and fixed.

7. Alternating-magnetic-field application device according to Claim 6, **characterized in that** the housing (28) has a length of approximately 65 mm to 70 mm, a height of approximately 20 mm and a width of approximately 35 mm, with an approximately oval cross section and rounded narrow sides,
**in that** the insertion tube (29) in comparison has a smaller diameter of approximately 10 mm and a length of approximately 70 mm to 100 mm.

8. Alternating-magnetic-field application device according to Claim 7, **characterized in that** the housing (28) and the insertion tube (29) are produced in a dimensionally stable fashion and at least the housing (28) has a coating made of a soft material, which adapts itself to the interior wall of the rectum (27).

9. Alternating-magnetic-field application device according to one of Claims 5 to 8, **characterized in that** the electrical connection lines (21) for the magnetic coil (20), and also a coolant inlet (30) and a coolant outlet (31), are connected to the insertion tube (29) and/or guided into the housing (28) through said tube.

10. Alternating-magnetic-field application device according to Claim 9, **characterized in that** the electrical connection lines (21) and coolant tubing (30) are held in a flexible connection tubing (32) which is attached to the insertion tube (29).

11. Alternating-magnetic-field application device according to Claim 10, **characterized in that** the electrical connection lines (21) and a coolant inlet tubing (30) are guided in the connection tubing (32) and in the insertion tube (29), and **in that** the remaining cross section (31) is used as a coolant return.

12. Alternating-magnetic-field application device according to one of Claims 1 to 11, **characterized in that** a field strength of approximately 3 kA/m to 4 kA/m is set on the large-scale applicator (1) and the active field concentrator brings about an approximately 3- to 4-fold increase in the field strength in the target volume, particularly in a diseased prostrate (23).

13. Alternating-magnetic-field application device according to one of Claims 1 to 11, **characterized in that** the active field concentrator (19), particularly when embodied as a rectal applicator (25), can be activated and used simultaneously or partly simultaneously with the large-scale applicator or can be activated and used independently and separately from the latter.

14. Alternating-magnetic-field application device according to one of Claims 1 to 13, **characterized in that** the device is designed for prefocussing the alternating magnetic field (12), particularly directly on the surface above and/or below a patient in the case of obese patients, by means of additional flat induction coils arranged thereon, which coils can be excited at a frequency and with a phase that is correspondingly synchronous with the alternating magnetic field (12) of the large-scale applicator (1).

## Revendications

1. Dispositif d'application de champ magnétique alternatif pour chauffer des substances magnétiques ou magnétisables dans un tissu biologique, en particulier pour la thermothérapie avec des nanoparticules magnétiques, présentant
un applicateur à large champ (1) avec une culasse magnétique (2) et deux pièces polaires (7, 8) situées en vis-à-vis sur la culasse magnétique (2) et séparées par un espace d'application de champ magnétique (13), et
avec deux bobines de champ magnétique (9, 10) respectivement associées à une pièce polaire (7, 8) avec une unité de commande d'applicateur à large champ pour l'alimentation en courant alternatif d'une amplitude donnée, d'une fréquence donnée et d'une position de phase donnée pour générer dans l'espace d'application (13) un champ magnétique alternatif (12) sensiblement homogène d'une intensité donnée,
le tissu biologique à exposer au champ magnétique pouvant être introduit dans l'espace d'application de champ magnétique (13) en tant que volume cible à exposer au champ magnétique, en particulier un patient avec une partie du corps à exposer au champ magnétique, telle une prostate pathologique (23), pouvant être placé dans l'espace d'application de champ magnétique (13),
**caractérisé en ce que** dans l'espace d'application de champ magnétique (13) à proximité du tissu biologique à exposer au champ magnétique en tant que volume cible d'exposition au champs magnétique, en particulier sur ou dans un patient à proximité d'une partie du corps à exposer champ magnétique, telle une prostate pathologique (23), un concentrateur de champ (19) est disposé, lequel concentre le champ magnétique alternatif (12) de l'applicateur à large champ (1) dans le volume cible en l'amplifiant ainsi localement à cet endroit, le concentrateur de champ étant de la ferrite en tant que concentrateur de champ passif, ou bien le concentrateur de champ étant un concentrateur de champ actif (19) avec au moins une bobine magnétique (20), et cette ou ces bobines magnétiques (20) étant orientées de manière à ce que les lignes du champ du concentrateur de champ actif (19) et de l'applicateur à large champ (1) soient essentiellement unidirectionnelles, ainsi qu'une unité de commande de concentrateur de champ (35, 38), avec laquelle cette ou ces bobines magnétiques (20) sont alimentées avec du courant alternatif synchronisé avec la même fréquence et la même phase de manière correspondante au courant alternatif de l'applicateur à large champ (1).

2. Dispositif d'application de champ magnétique alternatif selon la revendication 1, **caractérisé en ce que** l'unité de commande de l'applicateur à large champ et l'unité de commande du concentrateur de champ (38) pour un concentrateur de champ actif (19) sont reliées ensemble pour une synchronisation de la fréquence et de la position de phase ou bien sont intégrées l'une dans l'autre, moyennant quoi les amplitudes des courants alternatifs peuvent de préférence être réglées de manière indépendante respectivement pour l'applicateur à large champ et le concentrateur de champ.

3. Dispositif d'application de champ magnétique alternatif selon la revendication 1, **caractérisé en ce que** l'unité de commande du concentrateur de champ (38) en tant qu'unité indépendante de l'unité de commande de l'applicateur à large champ présente au moins un détecteur pour la détection et pour une synchronisation de la fréquence et de la position de phase avec le champ magnétique alternatif de l'applicateur à large champ (1).

4. Dispositif d'application de champ magnétique alternatif selon l'une quelconque des revendications 1 à 3, **caractérisé en ce que** cette ou ces bobines magnétiques du concentrateur de champ actif (19) est une bobine plate (20).

5. Dispositif d'application de champ magnétique alternatif selon l'une quelconque des revendications 1 à 4, **caractérisé en ce que** le concentrateur de champ actif (19) est réalisé en tant qu'applicateur rectal (25) avec un boîtier plat oblong (28) d'une forme et d'une taille adaptées au rectum (27) d'un patient en tant qu'espace de réception, et
**en ce que** le boîtier (28) contient une bobine plate (20) s'étendant longitudinalement de manière correspondante en guise de bobine magnétique, de sorte que la ligne de force du champ de celle-ci s'étend à peu près perpendiculairement au plan du boîtier.

6. Dispositif d'application de champ magnétique alternatif selon la revendication 5, **caractérisé en ce que**, sur une extrémité longitudinale du boîtier et de manière coudée par rapport au plan du boîtier, une tubulure d'introduction en forme de tuyau (29) est reliée au boîtier (28), moyennant quoi l'applicateur rectal (25) peut être introduit avec la tubulure d'introduction (29) à l'intérieur du rectum (27) en étant ainsi ajusté et fixé dans sa position.

7. Dispositif d'application de champ magnétique alternatif selon la revendication 6, **caractérisé en ce que** le boîtier (28) est d'une longueur d'environ 65 mm à 70 mm, d'une hauteur d'environ 20 mm et d'une largeur d'environ 35 mm avec une section transversale à peu près ovale et des surfaces étroites arrondies,
la tubulure d'introduction (29) présentant par contre un diamètre plus petit d'environ 10 mm ainsi qu'une longueur d'environ 70 mm à 100 mm.

8. Dispositif d'application de champ magnétique alternatif selon la revendication 7, **caractérisé en ce que** le boîtier (28) et la tubulure d'introduction (29) sont fabriqués de manière indéformable et **en ce qu'**au moins le boîtier (28) présente une surface de contact en un matériau tendre, laquelle s'adapte à la paroi intérieure du rectum (27).

9. Dispositif d'application de champ magnétique alternatif selon l'une quelconque des revendications 5 à 8, **caractérisé en ce que** les lignes de raccordement électrique (21) s'étendent vers la bobine magnétique (20) et **en ce qu'**une arrivée d'agent réfrigérant (30) et un écoulement d'agent réfrigérant (31) sont raccordés à la tubulure d'introduction (29) et/ou s'étendent par l'intermédiaire de celle-ci dans le boîtier (28).

10. Dispositif d'application de champ magnétique alternatif selon la revendication 9, **caractérisé en ce que** les lignes de raccordement électrique (21) et les tuyaux d'agent réfrigérant (30) sont réceptionnés dans un tuyau de raccordement flexible (32) monté sur la tubulure d'introduction (29).

11. Dispositif d'application de champ magnétique alternatif selon la revendication 10, **caractérisé en ce que** les lignes de raccordement électrique (21) et un tuyau d'arrivée d'agent réfrigérant (30) s'étendent dans le tuyau de raccordement (32) et dans la tubulure d'introduction (29) et **en ce que** la section transversale qui subsiste (31) est utilisée en tant que reflux d'agent réfrigérant.

12. Dispositif d'application de champ magnétique alternatif selon l'une quelconque des revendications 1 à 11, **caractérisé en ce que** l'on règle sur l'applicateur à large champ (1) une intensité de champ d'environ 3 kA/m à 4 kA/m et **en ce que** grâce au concentrateur de champ actif, on obtient une augmentation d'intensité du champ environ triple ou quadruple dans le volume cible, en particulier dans une prostate pathologique (23).

13. Dispositif d'application de champ magnétique alternatif selon l'une quelconque des revendications 1 à 11, **caractérisé en ce que** le concentrateur de champ actif (19) peut être utilisé en particulier en tant qu'applicateur rectal (25) de manière isochrone ou partiellement isochrone avec l'applicateur à large champ ou bien peut être activé et utilisé de manière indépendante et distincte de celui-ci.

14. Dispositif d'application de champ magnétique alternatif selon l'une quelconque des revendications 1 à 13, **caractérisé en ce que** le dispositif est étudié pour la préfocalisation du champ magnétique alternatif (12), en particulier en cas de patients à corps volumineux, directement à la surface au-dessus et/ou en-dessous d'un patient, grâce à d'autres bobines d'induction plates disposées à cet endroit, lesquelles peuvent être excitées en correspondance de manière synchrone en fréquence et en phase avec le champ magnétique alternatif (12) de l'applicateur à large champ (1).
